# EUROPEAN PATENT APPLICATION

(11) **EP 3 900 721 A1**
(43) Date of publication of application: **27.10.2021**
(21) Application number: 19875437.6
(22) Date of filing: 24.10.2019
(51) Int. Cl.: A61K 31/375, A61K 31/7088, A61P 17/00, A61P 17/14

(54) **COMPOSITION FOR ALLEVIATING AND TREATING HAIR LOSS, HAIR DAMAGE, AND SKIN DISEASE OF ANIMAL, COMPRISING APTAMIN C AS ACTIVE INGREDIENT**

(30) Priority: 25.10.2018 KR 20180128026
(71) Applicant: Nexmos Co., Ltd., Gyeonggi-do 16827 (KR)
(72) Inventor: SON, In Sik, Seongnam-Si Gyeonggi-do 13614 (KR)
(74) Representative: FRKelly
(86) International application number: PCT/KR2019/014029
(87) International publication number: WO 2020/085805

(57) **Abstract**

The invention relates to a composition for improving and treating hair loss, hair damage and skin diseases in animals comprising vitamin C and aptamer binding to the vitamin C as an active ingredient. As can be seen through the present invention, when a composition of the present invention comprising vitamin C and an aptamer binding to the vitamin C as an active ingredient is treated to hair loss, hair damage, atopic dermatitis, contact dermatitis, and eczema subjects, all of them show an improvement and therapeutic effect, and thus, there is an effect that can be used as a cosmetic and a drug for a subject with such a disease.

<110>     Nexmos Co., Ltd.

<120>     A COMPOSITION FOR IMPROVING AND TREATING ALOPECIA, HAIR DAMAGE,
          AND SKIN DISEASE OF ANIMAL COMPRISING APTAMIN C AS EFFECTIVE
          COMPONENT

<130>     OP19-0029HSPCT

<150>     KR 10-2018-0128026
<151>     2018-10-25

<160>     4

<170>     KopatentIn 2.0

<210>     1
<211>     32
<212>     DNA
<213>     Artificial Sequence

<220>
<223>     aptamer


<400>     1
gtggaggcgg tggccagtct catacgcggc ag                                     32


<210>     2
<211>     32
<212>     DNA
<213>     Artificial Sequence

<220>
<223>     aptamer


<400>     2
agagctcgcg ccggagttct caatgcaaga gc                                     32


<210>     3
<211>     32
<212>     DNA
<213>     Artificial Sequence

<220>
<223>     aptamer


<400>     3
gtggaggcgg tggccagtct cgcggtggcg gc                                     32


<210>     4
<211>     30
<212>     DNA
<213>     Artificial Sequence

<220>
<223>     aptamer

<400>    4
ggcacaacgg gcgcgcctcc atgctgttcg                                        30

## Description

### [Technical Field]

The present invention relates to a composition for improving and treating hair loss, hair damage and skin diseases in animals comprising vitamin C and an aptamer binding to the vitamin C as an active ingredient.

### [Background Art]

In general, the skin and hair of animals, especially companion animals, are parasitic with dandruff keratin, mites, demodex, and various other bacteria, which damage hair and cause hair loss, as well as frequently cause skin diseases. In addition, in general, the body of the companion animal often has an unpleasant odor peculiar to the companion animal. Therefore, in order to prevent the onset of companion animals and remove the peculiar odor of dogs, detergents or functional shampoos containing bactericidal ingredients were used.

However, since the detergent or functional shampoo has a functional limit because its sterilization effect and odor removal effect persistence is very insignificant, pets have to wash their companion animals frequently in order to prevent skin diseases of the companion animals.

In general, skin diseases that often occur in companion animals are as follows.

### (1) fungal skin disease

Fungal skin disease is a skin disease caused by the invasion of fungi (fungi) into the stratum corneum, hair and toenails of the skin. The main symptoms are hair loss or cracking due to rough hair. In addition, small dandruff substances are observed in the pet's fur, and the pet scratches or bites the infected area. Furthermore, when the itching becomes severe, there are cases where a wound is formed, which leads to bacterial infection. Fungal skin disease is known to be the most common skin disease in dogs, and it can infect humans, so pets must be quarantined and treated as soon as they are detected.

### (2) Nematode skin disease

Improved loyalty skin disease is a skin disease caused by external parasites commonly referred to as scabies or scavengers. The main symptom is partial hair loss, and gradually systemic hair loss is found, and bleeding occurs due to scratching or biting due to extreme itching (this is called itching). Most of the development sites are the tip of the ear, neck, armpit, and groin.

### (3) Eczema (dermatitis)

Eczema (dermatitis) is an itchy skin disease, the cause of which is uncertain and tends to recur, and its forms and patterns vary widely. In general, when referring to various eczema (dermatitis) collectively, it is called an eczematous skin disease group. Eczema is classified into acute and chronic. In acute cases, blisters (small blisters) are caught through papules (protruding the size of a millet or rice grain) and appear moist. On the other hand, in chronic cases, it looks dry and dry rather than moist. Unlike other skin diseases, eczema has a variety of types, but it is not difficult to treat and the treatment period is relatively short.

### (4) Seborrheic dermatitis

Seborrheic dermatitis belongs to eczema in a broad sense. It is generally referred to as seborrhea, and it occurs because the outermost skin layer, keratin, is not made normally due to epidermal detachment and sebum production. The main causes of seborrhea are endocrine hormonal abnormalities (hypothyroidism, genital dysfunction), fungal infections, ectoparasite infections (fleas, demodex, scabies, etc.), allergic substances (fleas, food, contact), environmental causes, autologous. Immune disease, malnutrition (protein, fat, vitamin A, zinc), etc. can be mentioned.

### (5) pyoderma

Pyoderma is a purulent disease of the skin that occurs when infected with bacteria. Bacteria involved in pyoderma include Staphylococci, Streptococci, Micrococcus, Pseudomonas aeruginosa, etc., and normal skin has a balance between normal bacteria and pathogenic bacteria to prevent the progression to pyoderma. Systemic antibiotic treatment and local sterilization treatment should be combined to remove the bacteria that cause the onset.

### (6) Allergic dermatitis

Like the human body, when foreign substances (antigens) such as bacteria or viruses enter the body of a pet, an antibody is created in response to it, forming a defense mechanism (immunity). However, when these defenses react excessively to contact with fleas, house dust mites, mosquito saliva, pollen, dust or wool, they release certain chemicals and cause a severe inflammatory reaction, which is called "allergy". What appears in is called "allergic dermatitis". Allergic dermatitis is a type of skin inflammation. With allergic dermatitis, pets may lick or scratch their ears, feet, legs, and groin, as well as hair loss symptoms.

### (7) Demodex dermatitis

Demodex canis occurs frequently in short-haired single seedlings, especially in bulldogs, dobelmanns, chihuahuas, and dachshunds. Demodex dermatitis develops when a pet becomes infected by exposure to the above-described Demodex. The main symptom is relatively less itching, but the symptoms of purulent disease or hair loss are severe, and the immune function is lowered, leading to local or systemic inflammation. Unlike other skin diseases, it takes a considerable period of time to treat, and in particular, it is often caused by the population of Demodex in the corners of the nose.

On the other hand, in order to treat skin diseases of pets, treatment was performed by receiving treatment at a pet hospital and administering expensive ointments, oral or injection drugs. However, due to the nature of skin diseases that require continuous treatment in the long term, excessive use of chemically synthesized drugs containing bactericidal components weakens immunity to the kidneys, liver, and skin tissues of pets. Even with the administration of the preparation and injection, the skin disease recurred and could not be cured. Furthermore, due to excessive use of chemical synthetic drugs for a long period of time, fatal damage to the liver or kidney tissue of the pet is caused, and as a result, there is a problem of causing complications due to weakened immunity due to weakened liver and kidney functions.

In order to treat skin diseases of pets as described above, it is necessary not only to bear a great economic burden, but also to continue attention to pets over a long period of time, and furthermore, in spite of such economic burdens and continuous interests, hair loss and hair damage of pets , It was not easy to cure skin diseases. As a result, some gave up treatment of pet hair loss, hair damage, and skin diseases, and even abandoned pets suffering from diseases.

### [Disclosure]

### [Technical Problem]

The present invention solves the above problems and is due to the necessity of the above, and an object of the present invention is to provide a composition for improving animal hair loss, hair damage, and skin diseases.

Another object of the present invention is to provide a composition for treating animal hair loss, hair damage, and skin diseases.

### [Technical Solution]

In order to achieve the above object, the present invention provides a composition for improving and treating hair loss, hair damage and skin diseases of animals comprising vitamin C and an aptamer that binds to the vitamin C as an active ingredient.

In one embodiment of the present invention, It is preferable that the skin disease is at least one disease selected from the group consisting of fungal skin disease, canine nematode dermatitis eczema, seborrheic dermatitis, pyoderma, allergic dermatitis, demodex dermatitis, atopic dermatitis, and contact dermatitis, but is not limited thereto.

In another embodiment of the present invention, the aptamer is preferably composed of one nucleotide sequence selected from the group consisting of nucleotide sequences of SEQ ID NOs: 1 to 4, but is not limited thereto.

In another embodiment of the present invention, the mixing ratio of vitamin C and aptamer of the composition is preferably in the range of 1:1 to 200:1 by weight, and it is more preferable that the mixing ratio of vitamin C and aptamer of the composition is in the range of 100:1 to 200:1 weight ratio, but is not limited thereto.

In another embodiment of the present invention, the animal is preferably a mammal, and a companion animal such as a dog or a cat is more preferable, but is not limited thereto.

In some embodiments, the concentration of aptamer in the pharmaceutically acceptable composition is between 0.001% and 2% and the concentration of vitamin C is between 0.1% and 20%.

In some embodiments, the pharmaceutically acceptable composition is a spray, liquid, ointment, cream, lotion, solution (e.g., aqueous solution), suspension, emulsion, paste, gel, powder, foam, slow release nanoparticle, slow release It is administered as a microparticle, bio-adhesive, patch, bandage, semi-solid dosage form or wound dressing.

In some embodiments, the pharmaceutically acceptable composition is administered once to twice daily.

Suitable formulation forms of the present invention may be provided in the form of, for example, solutions, gels, semi-solid or solid creams, skins, lotions, powders, ointments, sprays or conceal sticks. In addition, it may be prepared in the form of a foam or in the form of an aerosol composition further containing a compressed propellant.

In addition, the formulation composition of the present invention further comprises a fatty substance, a solubilizing agent, a thickening and gelling agent, an emollient, an antioxidant, a suspending agent, a stabilizer, a foaming agent, a fragrance, a surfactant, a water ionic or nonionic emulsifier, and a filler. , Sequestering and chelating agents, preservatives, vitamins, blockers, wetting agents, essential oils, dyes, pigments, hydrophilic or lipophilic actives, lipid vesicles or adjuvants commonly used in the field of dermatology. In addition, the above components may be introduced in an amount generally used in the field of dermatology. Products to which the formulation composition of the present invention can be added include, for example, lotions, essences, lotions, creams, packs, gels, ointments, patches or sprays.

### [Advantageous Effects]

As can be seen through the present invention, when a composition of the present invention comprising vitamin C and an aptamer binding to the vitamin C as an active ingredient is treated to hair loss, hair damage, atopic dermatitis, contact dermatitis, and eczema subjects, all of them show an improvement and therapeutic effect, and thus, there is an effect that can be used as a cosmetic and a drug for a subject with such a disease.

### [Description of Drawings]

Fig. 1 is a diagram showing the effect on contact dermatitis (Sequence),
Figs. 2 to 3 are pictures showing the effect on atopic dermatitis,
Fig. 4 is a picture showing the effect on skin diseases caused by Demodex infection,
Fig. 5 to 7 are pictures showing the effect on eczema,

In this drawing, aptamin C refers to an aptamer that binds to vitamin C (ascorbic acid).

### [Mode for Invention]

Hereinafter, the present invention will be described in detail with reference to Examples. Provided that, the following examples are for illustrative purposes only and are not intended to limit the scope of the present invention.

### Preparation Example: Preparation of the composition of the present invention

The aptamer sequence used in the present invention is shown in Table 1.

**[Table 1]**

| SEQ ID No | Sequence |
|---|---|
| 1 | GTGGA GGCGG TGGCC AGTCT CATAC GCGGC AG |
| 2 | AGAGC TCGCG CCGGA GTTCT CAATG CAAGA GC |
| 3 | GTGGA GGCGG TGGCC AGTCT CGCGG TGGCG GC |
| 4 | GGCAC AACGG GCGCG CCTCC ATGCT GTTCG |

The synthesis of the aptamer was performed by Integrated DNA Technologies (IDT). The synthesized aptamer and vitamin C (purchased from DSM) were prepared in a weight ratio described in each figure in distilled water at room temperature.

For reference, the aptamer sequences used in the experiments of the drawings of the present invention are shown in Table 2.

**[Table 2]**

| | |
|---|---|
| SEQ ID No 1 | Figs. 1, 2, 4 |
| SEQ ID No 2 | Figs. 3, 5 |
| SEQ ID No 3 | Figs. 6 |
| SEQ ID No 4 | Figs. 7 |

### Example 1: Effect on contact dermatitis

The composition of the present invention was prepared by mixing 0.2% (w/v) of ascorbic acid and 0.002% (w/v) of aptamer binding to vitamin C in distilled water.

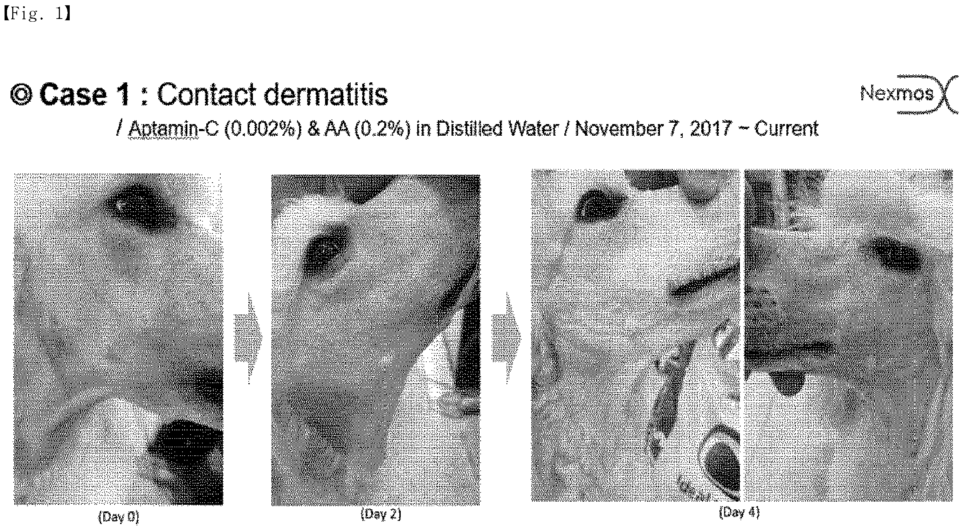

The prepared composition was applied to the affected area of a subject with contact skin disease, and as can be seen in FIG. 1, there was a slight difference depending on the severity of the subject's symptoms, but about 5 to 10 days after treatment Symptoms improved enough to be able to distinguish with the naked eye. In addition, no side effects were observed in them.

### Example 2: Effect on atopic dermatitis

The composition of the present invention was prepared by mixing 0.2% (w/v) of ascorbic acid and 0.002% (w/v) of aptamer binding to vitamin C in distilled water.

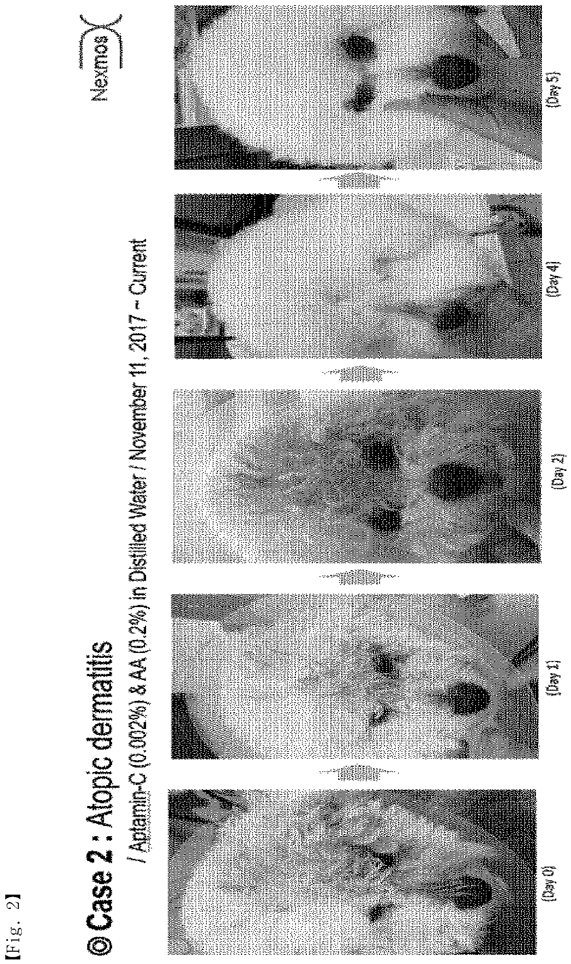

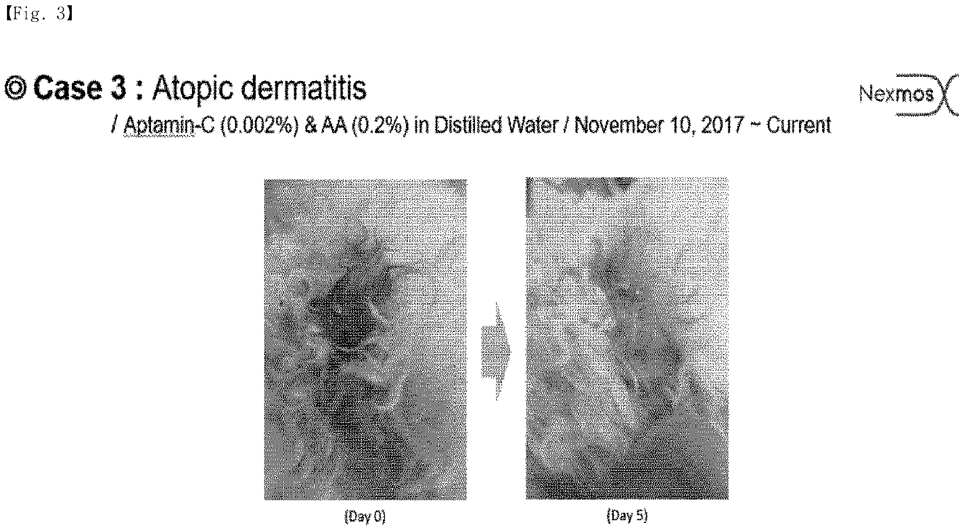

The prepared composition was applied to the affected area of a subject with atopic skin disease, and as can be seen in FIGs. 2 and 3, there was a slight difference depending on the severity of the subject's symptoms, but about 5 to 10 days after treatment Symptoms improved enough to be able to distinguish with the naked eye. In addition, no side effects were observed in them.

### Example 3: Effect on skin diseases caused by Demodex infection

The composition of the present invention was prepared by mixing 0.2% (w/v) of ascorbic acid and 0.002% (w/v) of aptamer binding to vitamin C in distilled water.

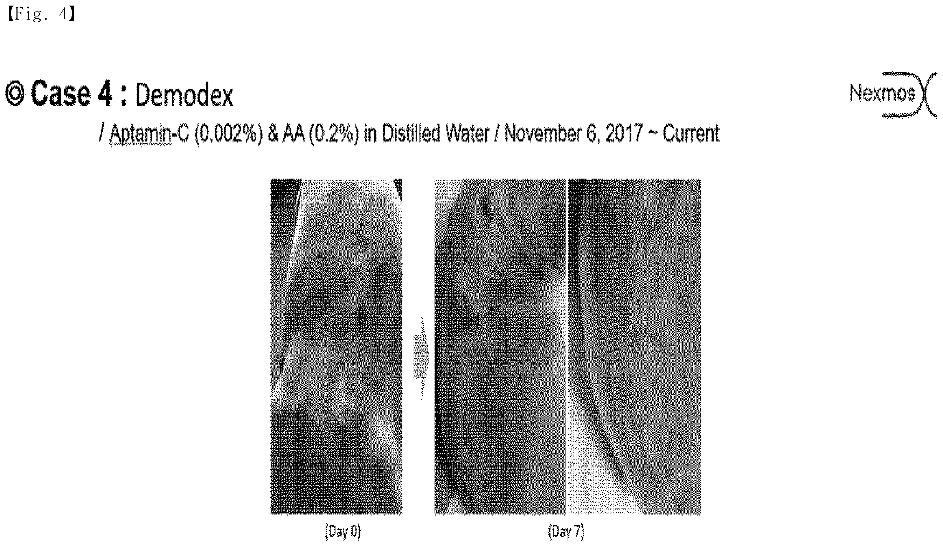

The prepared composition was applied to the affected area of a skin disease subject due to Demodex infection, and as can be seen in FIG. 4, there was a slight difference depending on the severity of the subject's symptoms, but about 5 to 10 days after treatment Symptoms improved enough to be able to distinguish with the naked eye. In addition, no side effects were observed in them.

### Example 4: Effect on eczema

The composition of the present invention was prepared by mixing 0.2% (w/v) of ascorbic acid and 0.002% (w/v) of aptamer binding to vitamin C in distilled water.

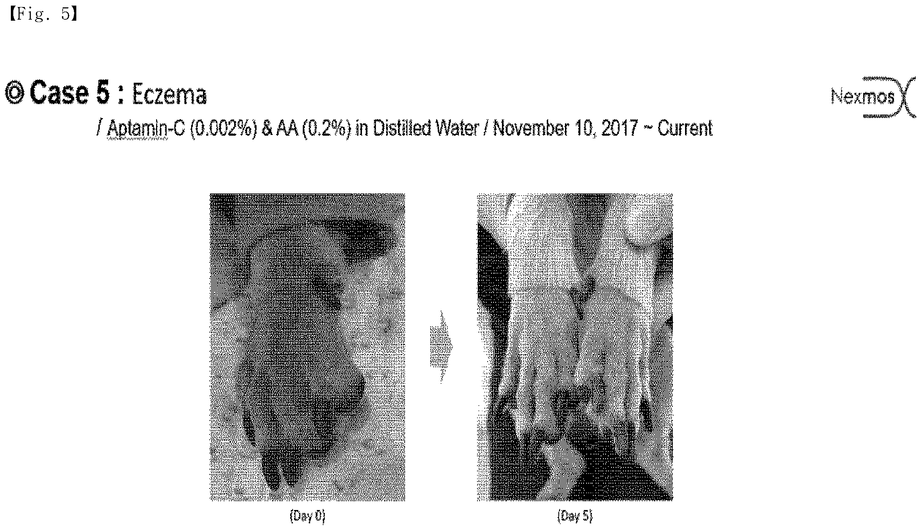

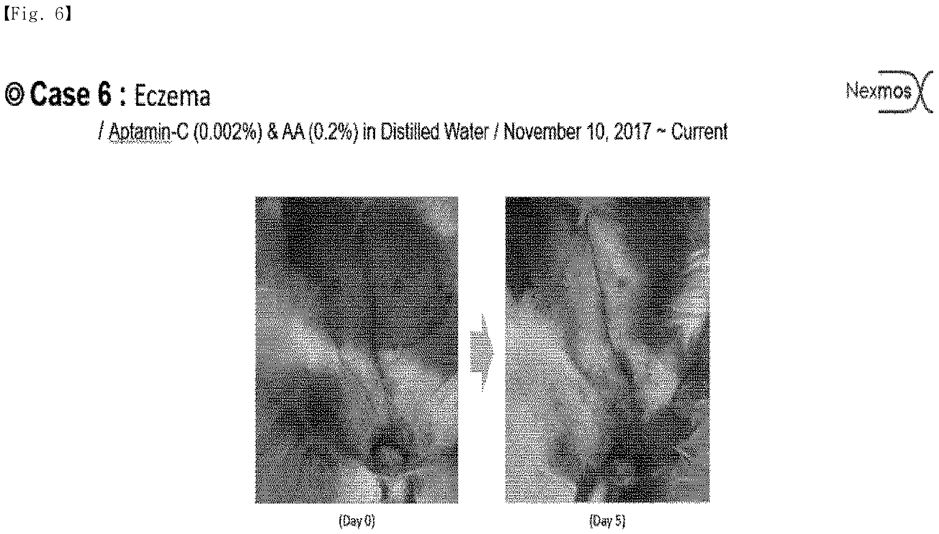

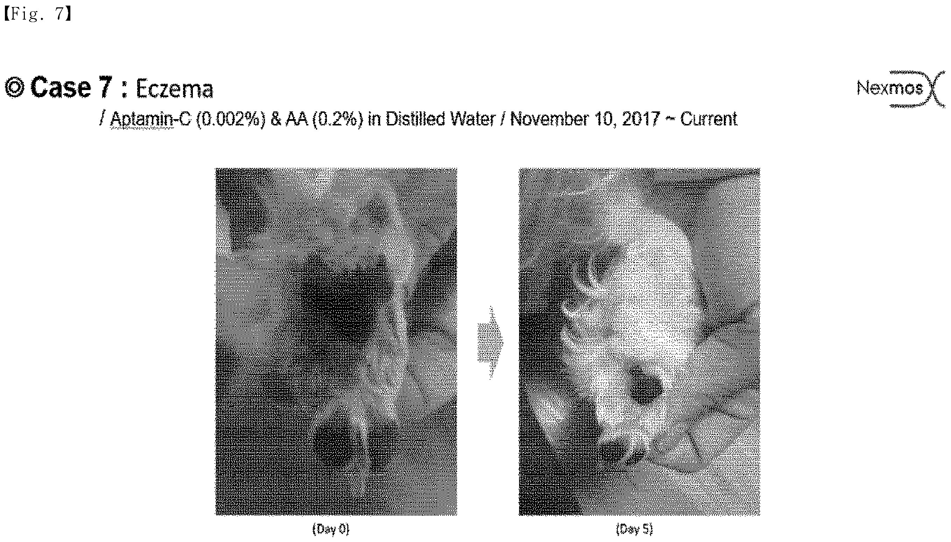

The prepared composition was applied to the affected area of a subject with eczema, and in all of them, as can be seen in Figs. 5 to 7, there was a slight difference depending on the severity of the subject's symptoms, but about 5 to 10 days after treatment Symptoms improved enough to be able to distinguish with the naked eye. In addition, no side effects were observed in them.

As can be seen in the drawings in common among the subjects of Examples 1 to 4, the effect of improving hair loss and hair damage was exhibited.

## Claims

1. A composition for improving and treating hair loss, hair damage and skin diseases in animals comprising vitamin C and aptamer binding to the vitamin C as an active ingredient.

2. The composition according to claim 1, wherein the skin disease is at least one disease selected from the group consisting of fungal dermatitis, canine nematode dermatitis, eczema, seborrheic dermatitis, pyoderma, allergic dermatitis, demodex dermatitis, atopic dermatitis, and contact dermatitis.

3. The composition according to claim 1, wherein the aptamer is selected from the group consisting of nucleotide sequences of SEQ ID NOs: 1 to 4.

4. The composition according to claim 1, wherein the mixing ratio of vitamin C and aptamer in the composition is in the range of 1:1 to 200:1 by weight.

5. The composition according to claim 1, wherein the animal is mammals.
